# EUROPEAN PATENT SPECIFICATION

(11) **EP 4 119 064 B1**
(45) Date of publication and mention of the grant of the patent: **09.04.2025**
(21) Application number: 22184571.2
(22) Date of filing: 13.07.2022
(51) Int. Cl.: A61B 17/12

(54) **EMBOLIC COIL PROXIMAL CONNECTING ELEMENT AND STRETCH RESISTANT FIBER**
PROXIMALES EMBOLIESPIRALVERBINDUNGSELEMENT UND DEHNUNGSBESTÄNDIGE FASER
ÉLÉMENT DE CONNEXION PROXIMAL DE BOBINE EMBOLIQUE ET FIBRE RÉSISTANT À L'ÉTIREMENT

(30) Priority: 14.07.2021 US 202117375482
(43) Date of publication of application: 18.01.2023
(73) Proprietor: DePuy Synthes Products, Inc., Raynham, MA 02767 (US)
(72) Inventor: LORENZO, Juan, Raynham, 02767 (US); MONTIDORO, Tyson, Raynham, 02767 (US); SOLAUN, Daniel, Raynham, 02767 (US); BLUMENSTYK, David, Raynham, 02767 (US)
(74) Representative: Carpmaels & Ransford LLP

(56) References cited:
- EP-A2- 3 760 139
- US-A1- 2011 092 997
- US-A1- 2017 105 739

## Description

### CROSS REFERENCE TO RELATED APPLICATION

This application is a continuation in part to prior filed U.S. Patent Application No. 16/573,469 filed on September 17, 2019.

### FIELD OF INVENTION

The present invention generally relates to implantable medical devices, and more particularly, to engagement features for mechanically releasably securing implantable medical devices to a delivery system.

### BACKGROUND

Aneurysms can be intravascularly treated by delivering a treatment device to the aneurysm to fill the sac of the aneurysm with embolic material and/or block the neck of the aneurysm to inhibit blood flow into the aneurysm. When filling the aneurysm sac, the embolic material can promote blood clotting to create a thrombotic mass within the aneurysm. When treating the aneurysm neck without substantially filling the aneurysm sac, blood flow into the neck of the aneurysm can be inhibited to induce venous stasis in the aneurysm and facilitate natural formation of a thrombotic mass within the aneurysm.

In some current treatments, multiple embolic coils are used to either fill the aneurysm sac or treat the entrance of the aneurysm neck. A common challenge among embolic coil treatments is that implanted coils and implanted portions of partially implanted coils can become entangled and difficult to reposition. In some instances, a physician may not be able to retract a partially implanted coil and may be forced to position the coil in a non-ideal location. Improperly positioning embolic coils at the aneurysm neck can potentially have the adverse effect of impeding the flow of blood in the adjoining blood vessel, particularly if the entrance and/or sac is overpacked. If a portion of the non-ideally implanted coil becomes dislodged, it can enter the neighboring blood vessel and promote clot formation, which can ultimately lead to an obstruction that is tethered to the aneurysm and therefor extremely difficult to treat. Conversely, if the entrance and/or sac is insufficiently packed, blood flow can persist into the aneurysm.

In some current treatments, an embolic coil is attached to a tubular delivery member and delivered via a delivery catheter to an aneurysm. During delivery, the embolic coil can be engaged to the delivery member's implant engagement/deployment system (referred to herein equivalently as an "engagement system" or "deployment system"). When the embolic coil is in position, the deployment system can release the coil, the coil can be left implanted, and the delivery member can be retracted. Some treatments utilize a mechanical engagement/deployment system that can be actuated by a physician to release the implant by pulling one or more wires or other elongated members referred to generically herein as a "pull wire".

Some of the challenges that have been associated with delivering and deploying embolic coils with delivery members having mechanical engagement systems include premature release of a coil and movement of the delivery member due to push back from densely packed treatment sites.

There is therefore a need for improved methods, devices, and systems to facilitate implantation of embolic coils and other implants facing similar challenges.

The disclosure of EP3760139A2 provides a delivery member that includes a flexible distal portion including a wound wire coil surrounded by a flexible sleeve and inhibited from extending lengthwise by a stretch resistant member positioned through the lumen of the coil. The delivery member can include hypotubes positioned on either side (distally and proximally) from the wound wire coil to which the stretch resistant member and the wound wire coil can be attached.

The disclosure of US2011/092997A1 provides a micro-coil assembly including: a micro-coil unit which is inserted into an cerebral aneurysm region of a patient; a coil pusher unit which is arranged adjacent to the micro-coil unit and carries the micro-coil unit to the cerebral aneurysm region of the patient; a tie which connects an end part of the micro-coil unit and the coil pusher unit; and a tensile wire which is relatively movably arranged in the coil pusher unit and coupled to the tie to tense and cut the tie when the micro-coil assembly is separated. The disclosure of US2017/105739A1 provides a mechanical system for delivery of implants that may include a detachment system for the release of a coil that deforms a detent to release (or eject) the implant.

### SUMMARY

It is an object of the present invention to provide systems and devices to meet the above-stated needs. In some examples presented herein, separation of coil windings within an embolic coil is reduced or prevented with a stretch resistant fiber that is positioned within the lumen of the coil. Reducing or preventing the separation of coil windings can in some cases prevent an implanted portion of a partially implanted coil from being tangled with implanted coils and thereby make it possible to more easily reposition and/or extract some or all of the coil. In some examples presented herein, during delivery of the embolic coil the distal end of the pull wire is supported by an engagement/detachment feature (referred to herein equivalently as "engagement feature", "detachment feature", or "key") affixed to the proximal end of the embolic coil. The support provided by the key can in some cases reduce the likelihood that the embolic coil is prematurely released. In some examples presented herein, the embolic implant can have a highly flexible proximal portion. The flexibility of the embolic implant can in some cases reduce the force on the delivery member due to push back from densely packed treatment sites and thereby reduce movement of the delivery member due to the push back.

The invention is set out in the appended independent claim 1. Further embodiments are set out in the dependent claims.

### BRIEF DESCRIPTION OF THE DRAWINGS

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
FIGs. 1A and 1B are illustrations of embolic implants;
FIGs. 2A and 2B are illustrations of detachment features each having a stretch resistant fiber therethrough;
FIG. 3 is an illustration of a stretch resistant fiber being inserted into a lumen of an embolic coil;
FIG. 4 is an illustration of a stretch resistant fiber exiting a lumen of an embolic coil;
FIGs. 5A and 5B are illustrations of detachment features being inserted into a lumen of an embolic coil;
FIGs. 6A and 6B are illustrations of detachment features affixed to an embolic coil;
FIG. 7 is an illustration of the stretch resistant fiber affixed to an end of the embolic coil;
FIGs. 8A through 8C illustrate a time sequence wherein an embolic coil stretches as a result of a non-optimal stretch resistant fiber placement;
FIG. 9 is an illustration of embolic coils being positioned within an aneurysm;
FIG. 10A is an illustration of embolic coils becoming tangled, and
FIG. 10B is an illustration of a tangled coil becoming elongated as illustrations of a problem with the prior art;
FIG. 11 is an illustration of a stretch resistant fiber inhibiting tangling and elongating of an embolic coil;
FIG. 12 is a flow diagram outlining method steps that can be conducted as part of an aneurysm treatment;
FIG. 13 is an illustration of an embolic implant secured to a delivery tube;
FIGs. 14A through 14D illustrate a sequence of steps for releasing an embolic implant from a delivery tube;
FIG. 15 is an illustration of an end of an embolic implant including a detachment feature expanding an inner diameter of an embolic coil;
FIG. 16 is an illustration of another example detachment feature according to aspects of the present invention;
FIGs. 17A through 17H are illustrations of example detachment features according to aspects of the present invention; and
FIG. 18 is an illustration of a proximal portion of the example detachment feature illustrated in FIG 16 according at aspects of the present invention.

### DETAILED DESCRIPTION

An object of the present invention is to attain more precise and repeatable implant detachment. More specifically, it is an object of the present invention to facilitate implantation of embolic coils and other implants facing challenges such as partially implanted implants becoming difficult to reposition, delivery systems shifting position due to push back during implantation, and/or implants releasing prematurely. To meet some or all of these needs, example implants can include a stretch resistant fiber to limit stretching and other deformation of the embolic portion (e.g. embolic coil) of the implant and a detachment feature to which the stretch resistant fiber can be secured and to which a delivery system can detachably attach.

To facilitate repositioning of the implant, the stretch resistant fiber can extend through the embolic coil and limit separation of windings of the coil when the coil is bent and pulled. By limiting the separation of the windings, the embolic coil is less likely to become tangled when partially implanted and less likely to be stretched or otherwise deformed when retracted when partially implanted. The embolic coil can thereby be more easily repositioned. In some examples, the detachment feature can include two separate openings, one for securing the stretch resistant fiber, and another for being engaged to an engagement system. The dual opening detachment feature can reduce potential manufacturing challenges to provide for reliable stretch resistant fiber positioning and therefore more reliably provide implants that can be more easily repositioned.

To reduce effects of push back during implantation, the detachment feature can be sized and affixed to the embolic coil to provide an embolic coil implant with a highly flexible proximal section. An embolic coil implant having a highly flexible proximal section can reduce push back force on the delivery tube and thereby mitigate the effects of the delivery tube shifting. Additionally, or alternatively, the detachment feature can be sized to mate with a delivery tube having a highly flexible distal section, and the highly flexible distal section of the delivery tube can mitigate the effects of the delivery tube shifting. When an embolic coil implant having a highly flexible proximal section is mated to a delivery tube having a highly flexible distal portion, the combination of the flexible distal section of the delivery tube and the flexible proximal section of the implant can further mitigate the effects of delivery tube shifting.

To reduce instances of premature deployment, the detachment feature can include a bridge to support a pull wire. The detachment feature can be detachably attached to a mechanical engagement/deployment system on a delivery tube. The detachment feature can include an opening through which a loop wire of a mechanical engagement system can pass. In some examples, the detachment feature can further include a bridge positioned distally from the opening on which a distal portion of the pull wire can rest. The bridge can inhibit the pull wire from deforming due to the engagement with the loop wire and can therefore reduce the likelihood that the implant is prematurely released due to bending of the pull wire.

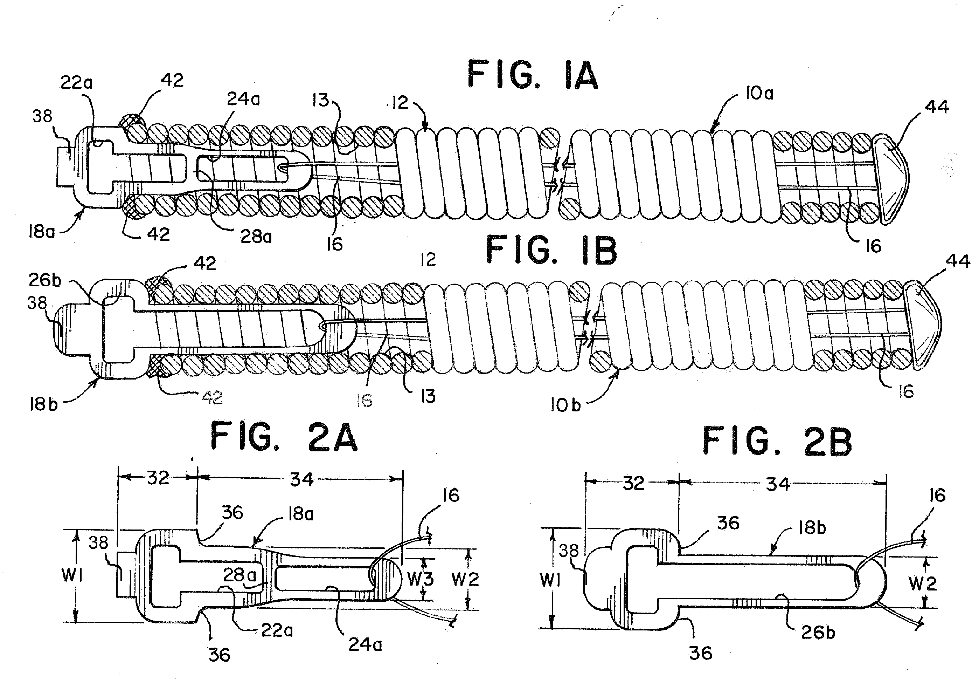

FIG. 1A is an illustration of an implant 10a including an embolic coil 12 with a lumen 13 therethrough, a detachment feature 18a, and a stretch resistant fiber 16. Portions of the coil 12 and welds 42 as illustrated in a cut-away view for the purposes of illustration. The detachment feature 18a can partially be positioned within the lumen 13 of the coil 12 and can extend out of the coil 12. The detachment feature 18a can include a distal opening 24a through which the stretch resistant wire 16 is looped, and a proximal opening 22a sized to receive a loop wire or other engagement mechanism of a mechanical implant engagement system. The detachment feature 18a can include a bridge 28a positioned between the distal opening 24a and the proximal opening 22a. The detachment feature 18a can include a proximal tab 38 sized to fit within a lumen of a delivery tube. The stretch resistant fiber 16 can be secured at an end of the embolic coil 12 opposite the end to which the detachment feature 18a is attached with a weld 44 or other appropriate attachment.

The detachment feature 18a can be tapered as it extends further within the lumen 13 of the embolic coil 12 to allow the embolic coil 12 to have additional flexibility where the embolic coil 12 surrounds the tapered region. The detachment feature 18a can also have a substantially flat profile, providing even greater flexibility in directions into and out of the plane of the image.

The detachment feature 18a can be sufficiently secured with attachments 42 to the coil 12 without fusing any windings of the coil 12 (as illustrated) or by fusing a small number of windings (e.g. 5 or fewer windings). Compared to known solutions wherein typically ten or more windings are soldered together (with limited control over the number of fused windings), the attachments 42 to the coil 12 can be realized with significantly fewer fused coil windings. By reducing the number of windings that are fused, the proximal section of the implant 10a can have increased flexibility compared to known designs which rely on fusing windings from the proximal end of the embolic coil.

FIG. 1B is an illustration of an alternatively constructed implant 10b having elements as described in relation to FIG. 1A with like reference numbers indicating like elements. Portions of the coil 12 and welds 42 as illustrated in a cut-away view for the purposes of illustration. Compared to the implant 10a illustrated in FIG. 1A, the implant 10b can have an alternative detachment feature 18b having a single opening 26b that provides an opening to which a mechanical engagement system can engage and through which the stretch resistant fiber 16 can be looped. The detachment feature 18b illustrated in FIG. 1B also lacks the extended tapered region of the detachment feature 18a illustrated in FIG. 1A. Although the tapered region of the detachment feature 18a illustrated in FIG. 1A can provide for a more flexible proximal section of the implant 10a compared to the implant 10b in FIG. 1B, the detachment feature 18b illustrated in FIG. 1B can nevertheless provide greater flexibility over known embolic coil implants by providing flexibility in directions into and out of the plane of the image by virtue of being flat and provide increased flexibility over designs which rely on fusing windings from the proximal end of the embolic coil by virtue of the low profile attachments 42.

FIGs. 2A and 2B through FIG. 7 illustrate a sequence of steps for constructing the implants 10a and 10b illustrated in FIGs. 1A and 1B. FIGs. 2A and 2B illustrate the stretch resistant fiber 16 being passed through the detachment features 18a, 18b. The detachment features 18a, 18b can be laser cut from a flat sheet material. The flat sheet material is preferably a radiopaque material that can be welded or otherwise affixed to the embolic coil 12.

FIG. 2A illustrates the dual opening detachment feature 18a having a proximal portion 32 that is sized to engage a mechanical engagement system and/or delivery tube. The proximal portion 32 is illustrated as having a width W1. The dual opening detachment feature 18a can have a distal portion 34 that is sized to fit within the lumen 13 of the embolic coil. The distal portion 34 can have a wider section having a width W2 that is about as wide as the inner diameter of the embolic coil 12 and a tapered section having a width W3 that is significantly narrower than the inner diameter of the embolic coil 12. The detachment feature 18a can have a proximal tab 38 that is narrower than the proximal portion 32 and is sized to fit within a lumen of a delivery tube.

FIG. 2B illustrates a single opening detachment feature 18b having a proximal portion 32 that is sized to engage a mechanical engagement system and/or delivery tube. The proximal portion 32 is illustrated having a width W1. The single opening detachment feature 18b can have a distal portion 34b narrower than the proximal portion 32 and sized to fit within the lumen 13 of the coil 12. The single opening detachment feature 18b can have a proximal tab 38 that is narrower than the proximal portion 32 and sized to fit within a lumen of a delivery tube.

After the detachment feature 18a, 18b is formed, the stretch resistant fiber 16 can be threaded through the distal opening 24a of the dual opening detachment feature 18a or the single opening 26b of the single opening detachment feature 18b.

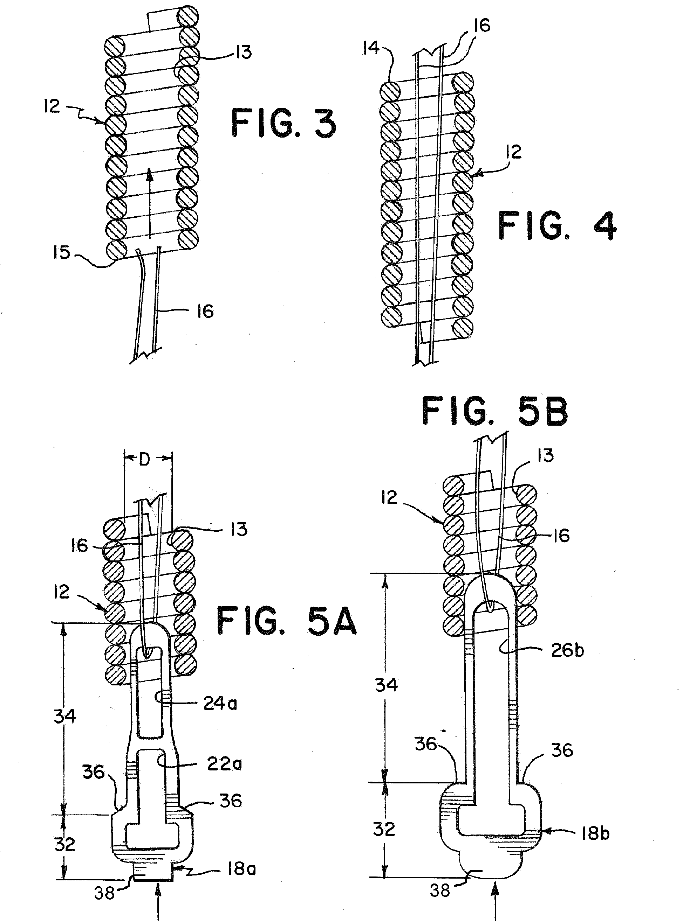

FIG. 3 is an illustration of the free ends of the stretch resistant fiber 16 being inserted into the proximal end 15 of the embolic coil 12. At the step illustrated in FIG. 3, the stretch resistant fiber 16 can be looped through a detachment feature 10a, 10b such as illustrated in FIGs. 2A and 2B.

FIG. 4 is an illustration of the free ends of the stretch resistant fiber 16 exiting the lumen 13 of an embolic coil 12 at the distal end 14 of the embolic coil 12.

FIGs. 5A and 5B are illustrations of detachment features 18a, 18b being inserted into the lumen 13 of an embolic coil 12. After exiting the distal end 14 of the embolic coil 12, the free ends of the stretch resistant fiber 16 can be further pulled as indicated by the arrow in FIG. 4 to move the detachment feature 18a, 18b into the lumen 13 of the embolic coil 12 at the proximal end 15 of the embolic coil 12 as illustrated in FIGs. 5A and 5B and as indicated by the arrows. Before entry of the detachment feature 18a, 18b into the lumen 13 of the embolic coil 12, the embolic coil can have an inner diameter D as indicated in FIG. 5A. The proximal portion 34 of the detachment feature 18a, 18b can be sized to have a width over at least a portion of the distal portion 34 that is about equal to the inner diameter D for a snug fit. Alternatively, or additionally, at least a portion of the distal portion 34 can have a width that is larger than the diameter D to create an interference fit. Alternatively, or additionally, at least a portion of the distal portion 34 can have a width that is smaller than the diameter D to allow for greater flexibility of the coil 12 near the proximal end 15 of the coil 12.

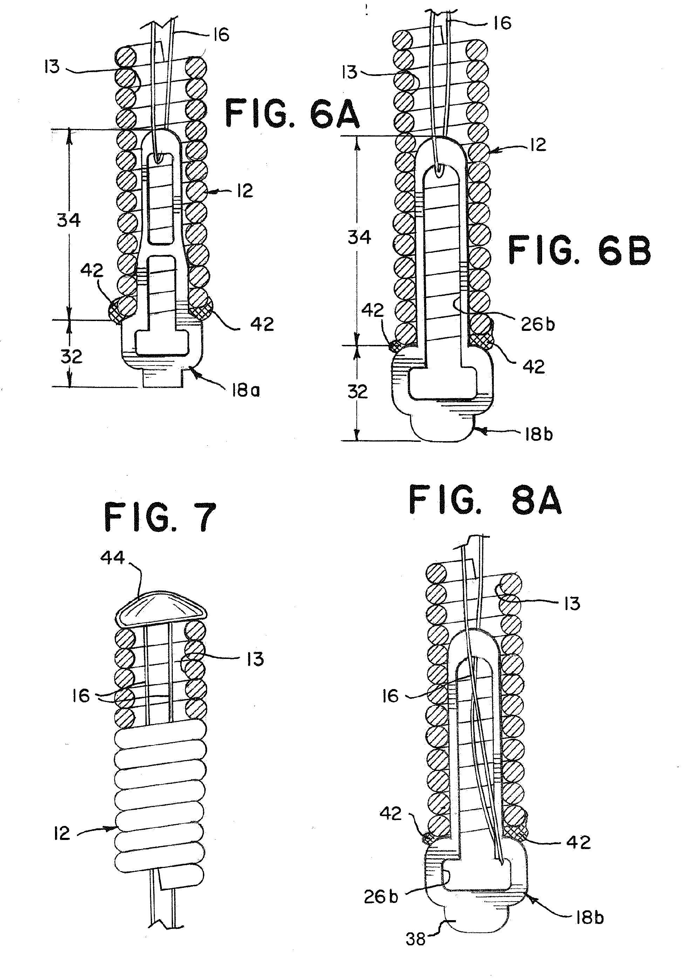

FIGs. 6A and 6B are illustrations of the detachment features 10a, 10b with the distal portion 34 fully inserted into the lumen 13 of the embolic coil 12 and the detachment feature 18a, 18b affixed to the embolic coil 12 with welds 42 or other attachment. In both FIGs. 6A and 6B, the detachment feature 18a, 18b is illustrated having a distal portion 34 that has a width over at least a portion of the length of the distal portion 34 that is about equal to the inner diameter D of the lumen 13 of the embolic coil 12.

FIG. 7 is an illustration of the stretch resistant fiber 16 affixed to the distal end of the embolic coil 12. After affixing the detachment feature 18a, 18b, or at least positioning the detachment feature 18a to 18b as illustrated in FIGs. 6A and 6B, the stretch resistant fiber 16 can be pulled tight to reduce slack in the fiber 16 and/or create tension in the fiber 16, and the fiber 16 can be affixed with a weld 44 or other attachment. After the fiber 16 is attached, the fiber can be substantially stretch resistant as to resist significant elongation due to forces applied to the embolic coil 12 during preparation for treatment, during delivery of the implant 10a, 10b, during positioning of the implant in a treatment site, during retraction of the implant, and during deployment of the implant. In other words, the stretch resistant fiber 16 can be effective to limit lengthening of the embolic coil 12 when the embolic coil 12 is retracted from an aneurysm, and the stretch resistant fiber 16 can be effective to limit separation of the windings within the embolic coil 12 when the embolic coil 12 is bent.

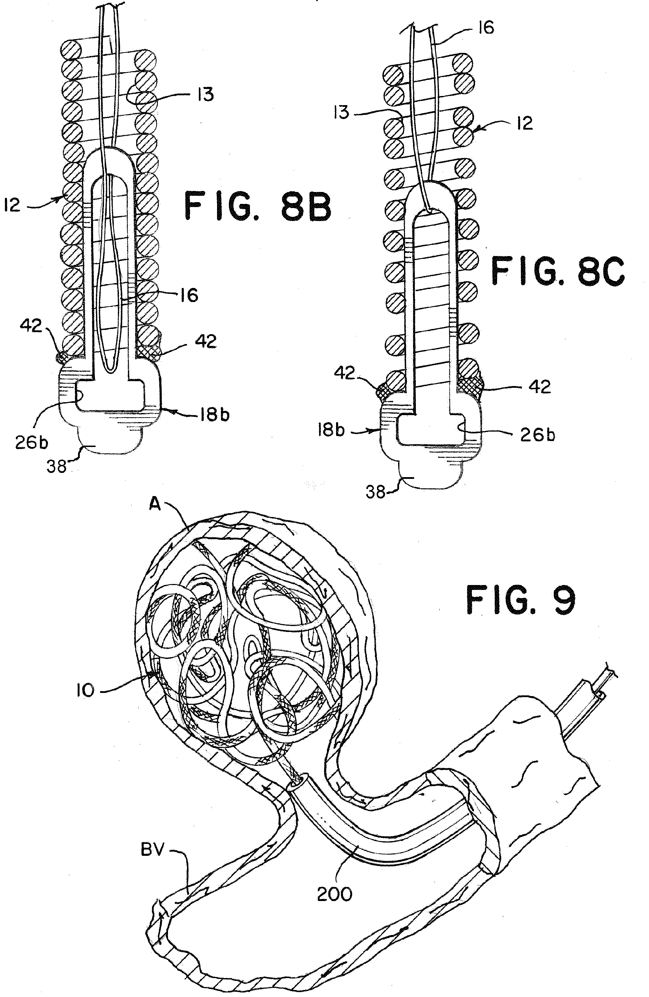

FIGs. 8A through 8C illustrate a time sequence wherein the embolic coil 12 is allowed to stretch as a result of a non-optimal stretch resistant fiber 16 placement. FIG. 8A illustrates a non-optimal fiber 16 placement within the single opening detachment feature 18b. The fiber 16 can become looped over a section of the detachment feature 18b that is not optimal such that movement of the fiber 16 as illustrated in FIG. 8B can cause the fiber 16 to disengage from the non-optimal position, and as illustrated in FIG. 8C, can allow the embolic coil 12 to stretch at least until the fiber 16 again becomes engaged to the detachment feature 18a. A manufacturing challenge is therefore to prevent the fiber 16 from being positioned at a non-optimal location such as illustrated in FIG. 8A when the attachment step illustrated in FIG. 7 is performed. If, after manufacturing is complete, the fiber 16 becomes dislodged from the non-optimal location as illustrated in FIG. 8B, when the implant 10b is manipulated, such as while being repositioned during a treatment, the embolic coil 12 can elongate as illustrated in FIG. 8C or otherwise deform.

An advantage of the dual opening detachment feature 18a is that the stretch resistant fiber 16 is less likely to become looped over a non-optimal section of the detachment feature 18a during manufacturing of the implant 10a illustrated in FIG. 1A.

FIG. 9 is an illustration of embolic implant(s) 10 being delivered through a delivery catheter 200 and positioned within an aneurysm A on a blood vessel BV. The implant(s) can loop and bend within the aneurysm sac to form a thrombotic mass. The implant(s) can loop back on themselves and/or loop next to other implants. As the aneurysm A becomes increasingly packed, overlapping portions of the implant 10 can press into each other.

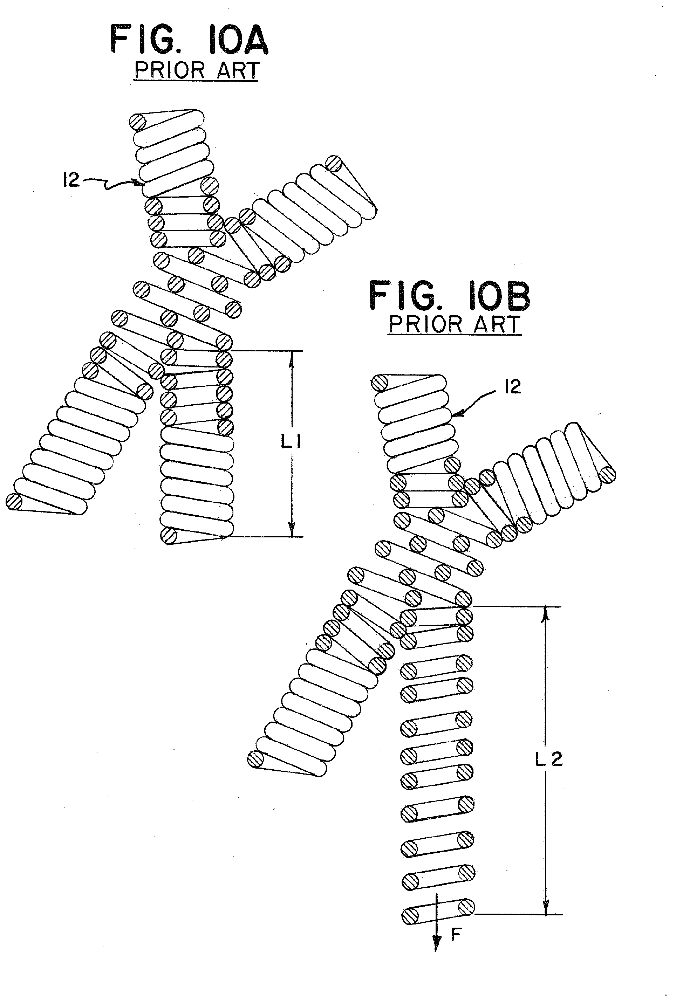

FIG. 10A is an illustration of embolic coils 12 that lack a stretch resistant fiber 16 becoming tangled as overlapping portions of the coils press into each other. This entanglement can make it difficult or impossible for either of the coils 12 to be repositioned, which is a known problem with some current embolic coil implants. FIG. 10B illustrates a portion of the embolic coil 12 becoming elongated to a length L2 that is longer than the length L1 of that section illustrated in FIG. 10A due to a force F. FIG. 10B illustrates a scenario wherein a physician may try to attempt to retract a tangled partially implanted embolic coil and may be not only unable to retract the coil but also exacerbate the already challenging treatment by now having to position the deformed elongated coil. Entanglement can become more likely when the windings of the embolic coil are separated, for example due to bending, or when the coils are more tightly pressed together due to dense packing.

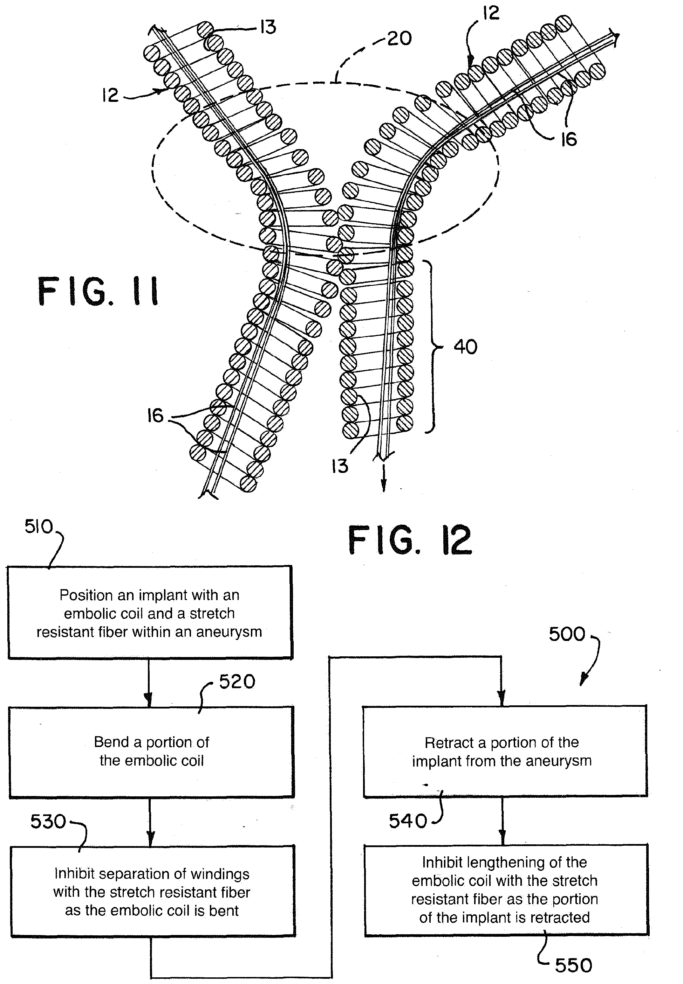

FIG. 11 is an illustration of example embolic coils 12 each having a stretch resistant fiber 16 being prevented from tangling and from elongating. Each coil 12 is illustrated as having a bent portion 20. The stretch resistant fiber 16 can shift within the lumen 13 of each coil to allow the coil 12 to flex and bend as needed when implanted. The fiber 16 can have sufficient tension to limit the amount of separation between windings in the bent portions 20. The separation of the winds can be so limited as to inhibit the windings of two adjacent coils 12 from becoming entangled as illustrated in FIG. 10A. FIG. 11 also illustrates the force F applied to a portion 40 of the coil 12 and the portion 40 being inhibited from elongating due to tension in the stretch resistant fiber 16. FIG. 11 illustrates a scenario wherein a physician may successfully retract a partially implanted embolic coil 12 having a stretch resistant fiber 16 therethrough.

FIG. 12 is a flow diagram illustrating a method 500 including steps that can be conducted as part of an aneurysm treatment using an example implant 10, 10a, 10b such as described herein. In step 510, an implant having an embolic coil and stretch resistant fiber can be positioned at least partially within an aneurysm sac. In step 520, a portion of the embolic coil can be bent. In step 530, as the coil is bent, the stretch resistant fiber can inhibit separation of windings within the bent portion of the embolic coil. In step 540, some or all of the implanted portion of the implant can be retracted from the aneurysm. In step 550, as the implant is retracted, the stretch resistant fiber can inhibit lengthening of the embolic coil.

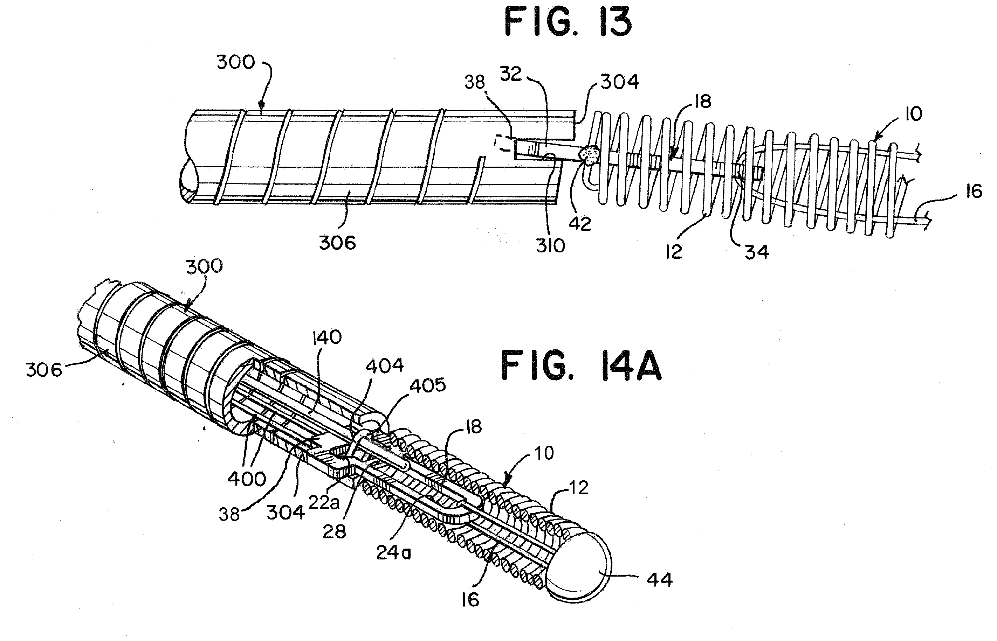

FIG. 13 is an illustration of an example embolic implant 10 such as either implant 10a, 10b illustrated in FIGs. 1A and 1B or otherwise described herein secured to a delivery tube 300. Example delivery tubes and engagement/deployment systems are described in U.S. Patent 10,806,461 and U.S. Patent 10,806,462. The delivery tube 300 can include a notch 310 sized to receive the proximal portion 32 of the detachment feature 18 of the implant 10, and likewise the proximal portion 32 of the detachment feature 18 can be sized to fit within the notch 310 of the delivery tube 300. FIG. 13 illustrates a side view of the implant 10 highlighting the flat profile of the detachment feature 18. As described in relation to FIGs. 1A and 1B, the implant 10 can have a highly flexible proximal section by virtue of the detachment feature 18 being flat and/or by virtue of the detachment feature 18 being secured to the coil 12 without fusing several coil windings. The detachment feature 18 can also be tapered for increased flexibility in directions into and out of the plane of the image. The detachment feature 18 can further include a proximal tab 38 positioned within the lumen of the delivery tube 300.

During an aneurysm occlusion treatment, lack of flexibility of the proximal section of known embolic implants and/or lack of flexibility of a distal portion of a delivery tube can cause the delivery tube to pull back from the treatment site or otherwise move out of position while the implant is being placed in the aneurysm. A delivery tube having a more flexible distal portion and an implant having a more flexible proximal section, alone or in combination, can therefore provide a more stable system for delivering the implant. Flexible structures, however, can tend deform or expand when manipulated. The stretch resistant fiber 16 and/or detachment feature 18 alone or in combination can support the coil 12 and inhibit deformation and expansion of the coil 12 according to the principles described herein. An object of the present invention is to provide an implant 10 having a highly flexible proximal section and/or configured to mate with a delivery tube 300 having a highly flexible distal portion.

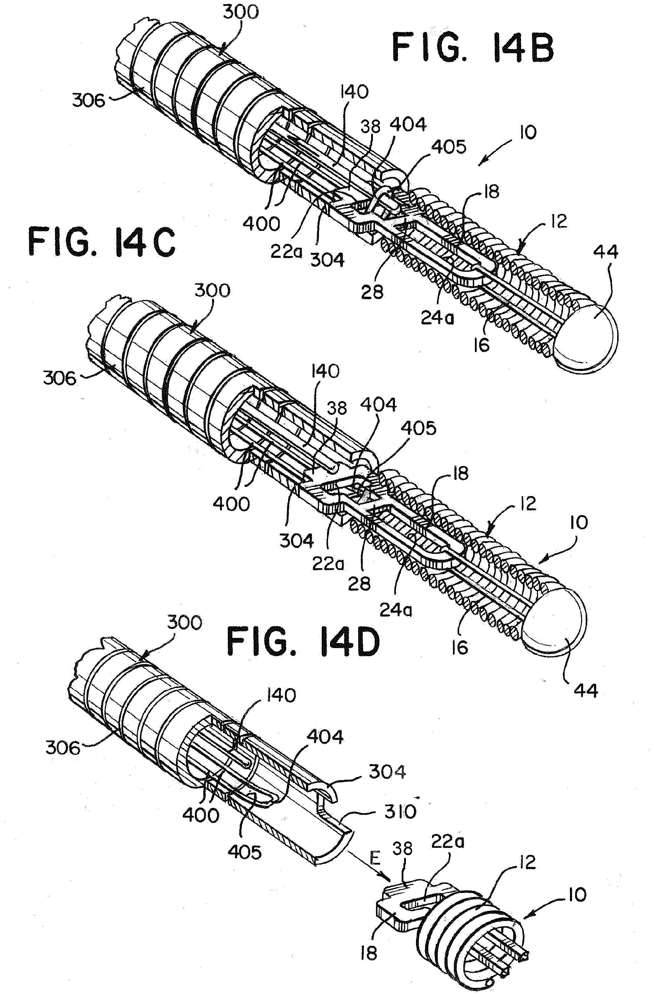

FIG. 14A is an illustration of the implant 10 and delivery tube 300 configured for delivery and positioning of the implant 10. FIGs. 14B through 14D are illustrations of a sequence of steps for releasing the example embolic implant 10 from the delivery tube 300. A portion of the delivery tube 300 is cut away for illustration purposes.

FIG. 14A illustrates the engagement system including a pull wire 140 and a loop wire 400 locked into the detachment feature 18 of the implant 12. The delivery tube 300 can include a compressible portion 306 that can be compressed. The loop wire 400 can have an opening 405 at a distal end 404 of the loop wire 400, and the opening 405 can be placed through an opening 22a in the detachment feature 18. When the pull wire 140 is put through the opening 405 the implant 12 is now secure.

The detachment feature 18 can include a bridge 28 positioned distally from the loop wire opening 405 and positioned to support a distal portion of the pull wire 140 that is distal of where the loop wire opening 405 is supported by the pull wire 140. Configured thusly, the bridge 28 can support the distal portion of the pull wire 140 such that when the loop wire 400 tugs on the pull wire 140 at the loop opening 405, the bridge 28 can inhibit the distal portion of the pull wire 140 from deforming. The proximal tab 38 can positioned to support a portion of the pull wire 140 that is proximal of where the loop wire opening 405 is supported by the pull wire 140. The combination of the bridge 28 and the proximal tab 38 can inhibit the pull wire 140 from deforming due to forces applied by the loop wire 400. The delivery tube 300 can be detachably attached to the implant 10 as illustrated in FIG. 14A during delivery of the implant 10 through the vasculature and while the implant 10 is being positioned at a treatment site. The bridge 28 can reduce the likelihood that the implant 10 is prematurely released due to bending of the pull wire 140 due to forces from the loop wire 400.

The bridge 28 can separate a proximal opening 22a and a distal opening 24a in a dual opening implant as illustrated. It is also contemplated that a single opening implant can be adapted to include a structure that can function to support the distal portion of the pull wire 140 similar to as described in relation to the illustrated bridge 28.

FIG. 14B illustrates the pull wire 140 being drawn proximally to begin the release sequence for the implant 10. FIG. 14C illustrates the instant the pull wire 140 exits the opening 405 and is pulled free of the loop wire 400. The distal end 404 of the loop wire 400 falls away and exits the locking portion 18. As can be seen, there is now nothing holding the implant 10 to the delivery tube 300. FIG. 14D illustrates the end of the release sequence. Here, the compressible portion 306 has expanded/returned to its original shape and "sprung" forward. An elastic force E is imparted by the distal end 304 of the delivery tube 300 to the medical device 10 to "push" it away to ensure a clean separation and delivery of the medical device 10.

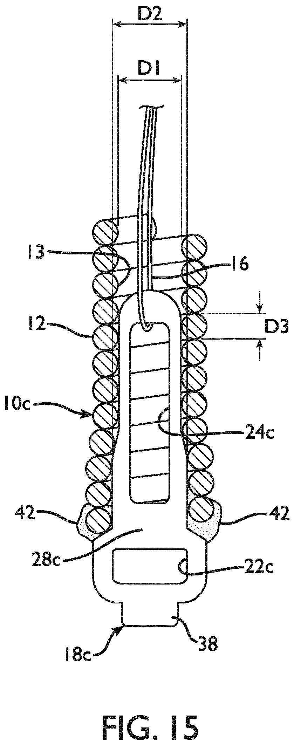

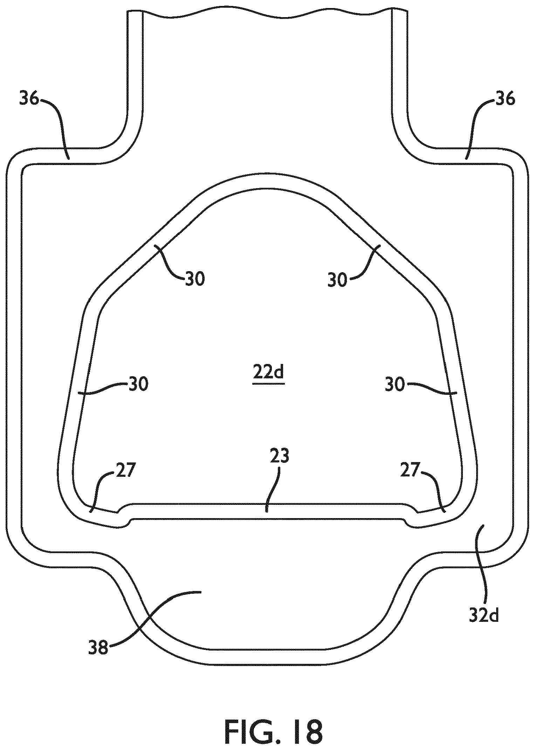

FIG. 15 is a cross sectional illustration of a proximal section of an alternatively constructed implant 10c having elements as described in relation to FIG. 1A with like reference numbers indicating like elements. Compared to the implant 10a illustrated in FIG. 1A, the implant 10c illustrated in FIG. 15 can have an alternative detachment feature 18c. The detachment feature 18c illustrated in FIG. 18c can have a portion with a width D2 sized to fit within a lumen 13 of an embolic coil 12 having an inner diameter D1. The width D2 of the detachment feature 18c can be larger than the inner diameter D1 of the coil lumen 13 so that when the detachment feature 18c is positioned within the lumen 13, a proximal portion of the lumen 13 expands to a diameter D2 to accommodate the width D2 of the detachment feature 18c. Configured thusly, the expanded portion of the coil 12 can provide a compressive force against the section of the detachment feature having width D2 to help secure the detachment feature 18c to the coil 12.

Compared to the implant 10a illustrated in FIG. 1A, the bridge 28c can extend proximally from a proximal end of the embolic coil 12. Configured thusly, in some configurations, the pull wire 140 need not be inserted into the lumen 13 of the embolic coil 12 to be supported by the bridge 28c. Limiting the length of pull wire 140 that is inserted into the embolic coil 12 can increase the flexibility of the proximal section of the embolic coil.

The implant 10c illustrated in FIG. 15 can be constructed according to the principles illustrated in FIGs. 2A and 2B through FIG. 7. The implant 10c illustrated in FIG. 15 can be used according to the principles illustrated in FIGs. 9 and 11 through 14D.

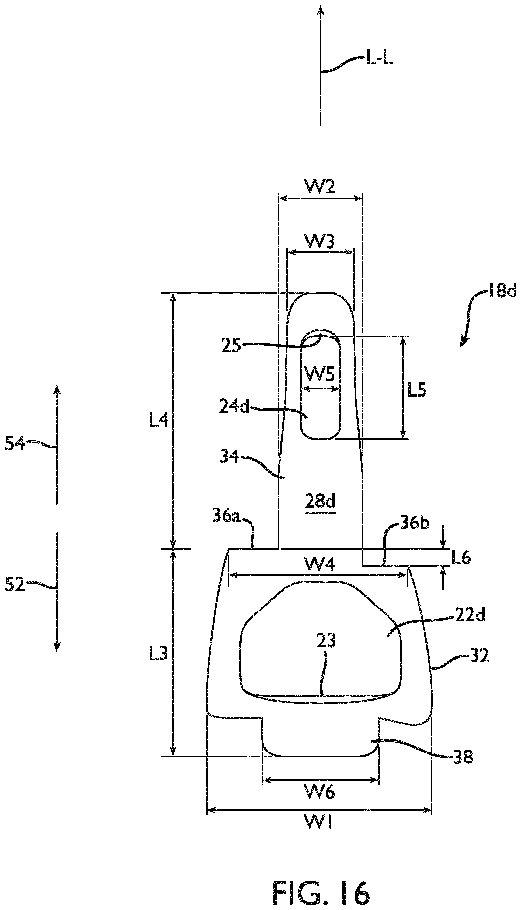

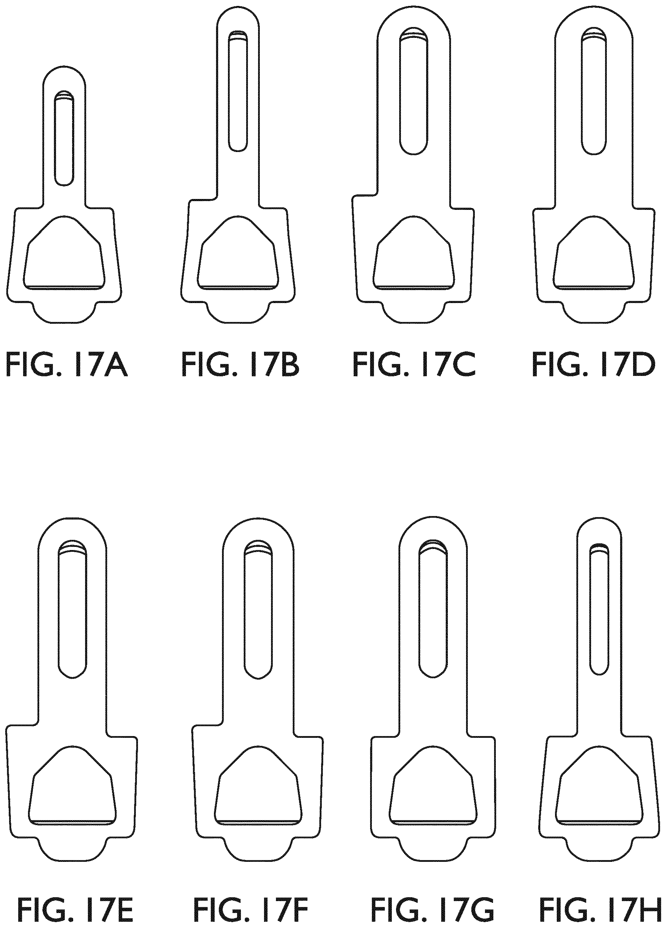

FIG. 16 is an illustration of another example detachment feature 18d. Various dimensions of the detachment feature 18d are illustrated in FIG. 16. The dimensions can be selected based on design criteria, so that the detachment feature 18d can be customized for a given implant. FIGs. 17A through 17H are illustrations of example detachment features having a similar general structure of the detachment feature 18d illustrated in FIG. 16 with the various dimensions illustrated in FIG. 16 adjusted to customize the detachment feature 18d. The detachment feature is oriented in relation to a longitudinal axis L-L, a distal direction 54, and a proximal direction 52.

The proximal portion 32 of the detachment feature 18d has a first width W1 near a proximal end of the detachment feature 18d that is sized to fit within notches 310 of the delivery tube 300. The first width W1 is preferably sized approximately equal to an outer diameter of the distal end 304 of the delivery tube 300.

The distal portion 34 of the detachment feature 18d has a second width W2 and a third width W3 similar to as disclosed in relation to FIGs. 2A and 15.

The proximal portion 32 of the detachment feature 18d has a fourth width W4 near engagement surfaces 36a, 36b of the detachment feature 18d. The fourth width W4 is preferably sized approximately equal to an outer diameter of the embolic coil 12.

A distal opening 24d of the detachment feature 18d has a fifth width W5. The fifth width W5 is sufficiently wide to allow insertion of the stretch resistant fiber 16 and narrow enough to allow for sufficient material of the distal portion 34 of the detachment feature 18d to maintain structural integrity.

The proximal extension 38 of the detachment feature 18d has a sixth width W6. The sixth width W6 is preferably sized about equal to, and less than a diameter of the lumen of the delivery tube 300 at the distal end 304 of the delivery tube 300.

The proximal portion 32 of the detachment feature 18d has a third length L3. The third length L3 is preferably sized about equal to, and greater than a depth of the notches 310 of the delivery tube 300.

The distal portion 34 of the detachment feature 18d has a fourth length L4. The fourth length L4 is preferably sized sufficiently long enough to facilitate assembly of the implant 10, maintain structural integrity of the implant 10, and provide sufficient material for the distal opening 24d and bridge 28d. The fourth length L4 is preferably sufficiently short to allow for flexibility of the proximal portion of the implant 10.

A distal opening 24d of the detachment feature 18d has a fifth length L5. The fifth length L5 is sufficiently long to allow insertion of the stretch resistant fiber 16 and short enough so that the fourth length L4 can be sufficiently short.

The detachment feature 18d can include a longitudinal offset between engagement surfaces 36a, 36b. The longitudinal offset (sixth length L6) is preferably sized approximately equal to one half a diameter D3 (FIG. 15) of the wire which winds to make the embolic coil 12.

The distal opening 24d can include an atraumatic surface 25 in contact with the stretch resistant fiber when the implant 10 is assembled. The atraumatic surface 25 can be shaped to reduce likelihood of abrasion of the stretch resistant fiber 16 by the detachment feature 18d. Likewise, the proximal opening 22d can include an atraumatic surface 23 shaped to reduce likelihood of abrasion of the loop wire 400 by the detachment feature 18d.

FIG. 18 is an illustration of the proximal portion 32d of the example detachment feature 18d illustrated in FIG 16. The proximal opening 22d is approximately a five sided polygon with a base having the atraumatic surface 23 and four, distally extending sides 30 which are approximately equal in length. Corners of the polygon are rounded. Corners 27 adjacent to the atraumatic surface 23 (polygon base) can be deepened to encourage the loop wire 400 to rest in the deepened corners 27.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%.

## Claims

1. An embolic implant (10) comprising:
an embolic coil (12) comprising a coiled wire;
a detachment feature (18, 18a, 18b, 18c, 18d) affixed to the embolic coil approximate a proximal end (15) of the embolic coil; and
a stretch resistant fiber (16) engaged to the detachment feature, extended through a lumen (13) of the embolic coil, and affixed to the embolic coil approximate a distal end (14) of the embolic coil;
**characterised in that** the detachment feature further comprises a first engagement surface (36, 36a, 36b) and a second engagement surface (36, 36a, 36b) longitudinally offset from each other by a length approximately equal to half of a diameter of the coiled wire.

2. The embolic implant of claim 1,
wherein the detachment feature comprises a first opening (24a, 24c, 24d) therethrough and a second opening (22a, 22c, 22d) therethrough separated from the first opening,
wherein the stretch resistant fiber passes through the first opening,
wherein at least a portion of the first opening is positioned within the lumen of the embolic coil, and
wherein at least a portion of the second opening is positioned proximally from the proximal end of the embolic coil.

3. The embolic implant of claim 2, wherein the second opening comprises an atraumatic surface (23) at a proximal end of the second opening.

4. The embolic implant of claim 2, wherein the first opening comprises an atraumatic surface (25) at a distal end of the first opening.

5. The embolic implant of claim 2, wherein the first opening comprises an atraumatic surface (25) orthogonal to a longitudinal axis of the detachment feature.

6. The embolic implant of claim 2, wherein the second opening comprises an approximately polygon shape comprising four sides (30) of approximately equal length and a fifth side of greater length than the four sides, the fifth side being orthogonal to a longitudinal axis of the detachment feature, optionally wherein the polygon shape comprises two corners (27) on either end of the fifth side that are each respectively extended in a proximal direction in relation to the fifth side.

7. The embolic implant of claim 1,
wherein the lumen of the embolic coil comprises an inner diameter,
wherein the detachment feature comprises proximal portion (32d) disposed proximally from the lumen and a distal portion (34, 34b) disposed within the lumen,
wherein the proximal portion comprises a first width measuring greater than the inner diameter of the lumen, and
wherein the distal portion comprises a second width measuring about equal to the inner diameter of the lumen.

8. A system comprising:
the embolic implant of claim 1, the detachment feature comprising a distal portion (34, 34b) extended within a lumen (13) of the embolic coil and a proximal portion (32d) extending proximally from a proximal end (15) of the embolic coil, wherein the first engagement surface is affixed to the proximal end of the embolic coil, and wherein the second engagement surface is affixed to the proximal end of the embolic coil;
a pull wire (140); and
a delivery tube (300) attached to the detachment feature and configured to detach from the implant upon proximal translation of the pull wire.

9. The system of claim 8, wherein the detachment feature further comprises a distal opening (24a, 24c, 24d) through the distal portion, and
wherein the stretch resistant fiber extends through the distal opening.

10. The system of claim 9, wherein the distal opening comprises an atraumatic surface (25) apposed to the stretch resistant fiber.

11. The system of claim 8, further comprising:
a loop wire (400) affixed to the delivery tube,
wherein the detachment feature further comprises a proximal opening (22a, 22c, 22d) through the proximal portion, and
wherein the loop wire is extended through the proximal opening and around the pull wire so that the delivery tube is attached to the detachment feature.

12. The system of claim 11, wherein the proximal opening comprises an atraumatic surface (23) apposed to the loop wire, and optionally:
wherein the atraumatic surface comprises two deepened corners (27) each respectively extended in a proximal direction in relation to side of the atraumatic opening that is orthogonal to a longitudinal axis of the detachment feature, and
wherein the loop wire is positioned within each of the two deepened corners.

13. The system of claim 11, wherein the proximal opening is shaped to approximate a five sided polygon.

14. The system of claim 8,
wherein the detachment feature further comprises a distal opening (24a, 24c, 24d) through the distal portion,
wherein the detachment feature further comprises a proximal opening (22a, 22c, 22d) through the proximal portion,
wherein the detachment feature further comprises a bridge (28, 28a, 28c, 28d) separating the distal opening and the proximal opening, and
wherein the bridge supports a portion of the pull wire.

15. The system of claim 8, wherein (i) the proximal portion of the detachment feature comprises a width approximately equal to an outer diameter of the delivery tube, (ii) the proximal portion of the detachment feature comprises a width approximately equal to an outer diameter of the embolic coil, or (iii) the proximal portion comprises a proximal extension (38) comprising a width less than and approximately equal to a diameter of a lumen of the delivery tube at a distal end of the delivery tube.

## Patentansprüche

1. Embolisches Implantat (10), umfassend:
eine embolische Spule (12), umfassend einen gewickelten Draht;
ein Ablösemerkmal (18, 18a, 18b, 18c, 18d), das an der embolischen Spule in der Nähe eines proximalen Endes (15) der embolischen Spule befestigt ist; und
eine dehnungsbeständige Faser (16), die mit dem Ablösemerkmal in Eingriff gebracht ist, das sich durch ein Lumen (13) der embolischen Spule erstreckt und an der embolischen Spule in der Nähe des distalen Endes (14) der embolischen Spule befestigt ist;
**dadurch gekennzeichnet, dass** die Ablösevorrichtung ferner eine erste Eingriffsfläche (36, 36a, 36b) und eine zweite Eingriffsfläche (36, 36a, 36b) umfasst, die in Längsrichtung um eine Länge voneinander versetzt sind, die ungefähr der Hälfte eines Durchmessers des gewickelten Drahtes entspricht.

2. Embolisches Implantat nach Anspruch 1,
wobei das Ablösemerkmal eine erste Öffnung (24a, 24c, 24d) da hindurch und eine zweite Öffnung (22a, 22c, 22d) da hindurch, die von der ersten Öffnung getrennt ist, umfasst,
wobei die dehnungsbeständige Faser durch die erste Öffnung verläuft,
wobei mindestens ein Abschnitt der ersten Öffnung innerhalb des Lumens der embolischen Spule positioniert ist, und
wobei mindestens ein Abschnitt der zweiten Öffnung proximal von dem proximalen Ende der embolischen Spule positioniert ist.

3. Embolisches Implantat nach Anspruch 2, wobei die zweite Öffnung eine atraumatische Oberfläche (23) an einem proximalen Ende der zweiten Öffnung umfasst.

4. Embolisches Implantat nach Anspruch 2, wobei die erste Öffnung eine atraumatische Oberfläche (25) an einem distalen Ende der ersten Öffnung umfasst.

5. Embolisches Implantat nach Anspruch 2, wobei die erste Öffnung eine atraumatische Oberfläche (25) umfasst, die orthogonal zu einer Längsachse des Ablösemerkmals ist.

6. Embolisches Implantat nach Anspruch 2, wobei die zweite Öffnung eine annähernd polygonale Form aufweist, die vier Seiten (30) von annähernd gleicher Länge und eine fünfte Seite von größerer Länge als die vier Seiten umfasst, wobei die fünfte Seite orthogonal zu einer Längsachse des Ablösemerkmals ist, wobei optional die polygonale Form zwei Ecken (27) an jedem Ende der fünften Seite umfasst, die jeweils in einer proximalen Richtung in Bezug auf die fünfte Seite verlängert sind.

7. Embolisches Implantat nach Anspruch 1,
wobei das Lumen der embolischen Spule einen Innendurchmesser umfasst,
wobei das Ablösemerkmal einen proximalen Abschnitt (32d), der proximal von dem Lumen angeordnet ist, und einen distalen Abschnitt (34), 34b, der innerhalb des Lumens angeordnet ist, umfasst,
wobei der proximale Abschnitt eine erste Breite umfasst, die größer als der Innendurchmesser des Lumens ist, und
wobei der distale Abschnitt eine zweite Breite umfasst, die dem Innendurchmesser des Lumens etwa gleich ist.

8. System, umfassend:
das embolische Implantat nach Anspruch 1, wobei das Ablösemerkmal einen distalen Abschnitt (34, 34b), der sich innerhalb eines Lumens (13) der embolischen Spirale erstreckt, und einen proximalen Abschnitt (32d), der sich proximal von einem proximalen Ende (15) der embolischen Spirale erstreckt, umfasst, wobei die erste Eingriffsfläche an dem proximalen Ende der embolischen Spirale befestigt ist und wobei die zweite Eingriffsfläche an dem proximalen Ende der embolischen Spirale befestigt ist;
einen Zugdraht (140); und
ein Zufuhrrohr (300), das an dem Ablösemerkmal angebracht und so konfiguriert ist, dass es sich bei proximaler Verschiebung des Zugdrahtes von dem Implantat löst.

9. System nach Anspruch 8, wobei das Ablösemerkmal ferner eine distale Öffnung (24a, 24c, 24d) durch den distalen Abschnitt umfasst, und
wobei die dehnungsbeständige Faser sich durch die distale Öffnung erstreckt.

10. System nach Anspruch 9, wobei die distale Öffnung eine atraumatische Oberfläche (25) aufweist, die der dehnungsbeständigen Faser gegenüberliegt.

11. System nach Anspruch 8, ferner umfassend:
einen Schleifendraht (400), der an dem Zufuhrrohr befestigt ist,
wobei das Ablösemerkmal ferner eine proximale Öffnung (22a, 22c, 22d) durch den proximalen Abschnitt umfasst, und
wobei sich der Schleifendraht durch die proximale Öffnung und um den Zugdraht herum erstreckt, so dass das Abgaberohr an dem Ablösungsmerkmal befestigt ist.

12. System nach Anspruch 11, wobei die proximale Öffnung eine atraumatische Oberfläche (23) umfasst, die dem Schleifendraht gegenüberliegt, und optional:
wobei die atraumatische Oberfläche zwei vertiefte Ecken (27) umfasst, die sich jeweils in einer proximalen Richtung in Bezug auf die Seite der atraumatischen Öffnung erstrecken, die orthogonal zu einer Längsachse des Ablösemerkmals ist, und
wobei der Schleifendraht jeweils innerhalb der beiden vertieften Ecken positioniert ist.

13. System nach Anspruch 11, wobei die proximale Öffnung eine Form aufweist, die annähernd einem fünfseitigen Polygon entspricht.

14. System nach Anspruch 8,
wobei die Ablösevorrichtung ferner eine distale Öffnung (24a, 24c, 24d) durch den distalen Abschnitt umfasst,
wobei das Ablösemerkmal ferner eine proximale Öffnung (22a, 22c, 22d) durch den proximalen Abschnitt umfasst,
wobei das Ablösemerkmal ferner eine Brücke (28, 28a, 28c, 28d) umfasst, die die distale Öffnung und die proximale Öffnung trennt, und
wobei die Brücke einen Abschnitt des Zugdrahtes stützt.

15. System nach Anspruch 8, wobei (i) der proximale Abschnitt des Ablösemerkmals eine Breite aufweist, die annähernd gleich einem Außendurchmesser des Zufuhrrohrs ist, (ii) der proximale Abschnitt des Ablösemerkmals eine Breite aufweist, die annähernd gleich einem Außendurchmesser der embolischen Spule ist, oder (iii) der proximale Abschnitt eine proximale Verlängerung (38) aufweist, die eine Breite aufweist, die kleiner als und annähernd gleich einem Durchmesser eines Lumens des Zufuhrrohrs an einem distalen Ende des Zufuhrrohrs ist.

## Revendications

1. Implant pour embolisation (10) comprenant :
une spire métallique pour embolisation (12) comprenant un fil enroulé ;
un élément de détachement (18, 18a, 18b, 18c, 18d) fixé à la spire métallique pour embolisation à proximité d'une extrémité proximale (15) de la spire métallique pour embolisation ; et
une fibre résistante à l'étirement (16) mise en prise avec l'élément de détachement, étendue à travers une lumière (13) de la spire métallique pour embolisation, et fixée à la spire métallique pour embolisation à proximité d'une extrémité distale (14) de la spire métallique pour embolisation ;
**caractérisé en ce que** l'élément de détachement comprend en outre une première surface de prise (36, 36a, 36b) et une seconde surface de prise (36, 36a, 36b) décalées longitudinalement l'une de l'autre d'une longueur approximativement égale à la moitié d'un diamètre du fil enroulé.

2. Implant pour embolisation selon la revendication 1,
dans lequel l'élément de détachement comprend une première ouverture (24a, 24c, 24d) à travers celui-ci et une seconde ouverture (22a, 22c, 22d) à travers celui-ci distincte de la première ouverture,
dans lequel la fibre résistante à l'étirement passe à travers la première ouverture,
dans lequel au moins une partie de la première ouverture est positionnée à l'intérieur de la lumière de la spire métallique pour embolisation, et
dans lequel au moins une partie de la seconde ouverture est positionnée à proximité de l'extrémité proximale de la spire métallique pour embolisation.

3. Implant pour embolisation selon la revendication 2, dans lequel la seconde ouverture comprend une surface atraumatique (23) au niveau d'une extrémité proximale de la seconde ouverture.

4. Implant pour embolisation selon la revendication 2, dans lequel la première ouverture comprend une surface atraumatique (25) au niveau d'une extrémité distale de la première ouverture.

5. Implant pour embolisation selon la revendication 2, dans lequel la première ouverture comprend une surface atraumatique (25) orthogonale à un axe longitudinal de l'élément de détachement.

6. Implant pour embolisation selon la revendication 2, dans lequel la seconde ouverture comprend une forme approximativement polygonale comprenant quatre côtés (30) de longueur approximativement égale et un cinquième côté de longueur supérieure aux quatre côtés, le cinquième côté étant orthogonal à un axe longitudinal de l'élément de détachement, éventuellement dans lequel la forme polygonale comprend deux coins (27) sur chaque extrémité du cinquième côté qui sont chacun respectivement étendus dans une direction proximale par rapport au cinquième côté.

7. Implant pour embolisation selon la revendication 1,
dans lequel la lumière de la spire métallique pour embolisation comprend un diamètre interne,
dans lequel l'élément de détachement comprend une partie proximale (32d) disposée à proximité de la lumière et une partie distale (34, 34b) disposée à l'intérieur de la lumière,
dans lequel la partie proximale comprend une première largeur supérieure au diamètre interne de la lumière, et
dans lequel la partie distale comprend une seconde largeur environ égale au diamètre interne de la lumière.

8. Système comprenant :
l'implant pour embolisation selon revendication 1, l'élément de détachement comprenant une partie distale (34, 34b) étendue à l'intérieur d'une lumière (13) de la spire métallique pour embolisation et une partie proximale (32d) s'étendant à proximité d'une extrémité proximale (15) de la spire métallique pour embolisation, dans lequel la première surface de prise est fixée à l'extrémité proximale de la spire métallique pour embolisation, et dans lequel la seconde surface de prise est fixée à l'extrémité proximale de la spire métallique pour embolisation ;
un fil de traction (140) ; et
un tube de pose (300) attaché à l'élément de détachement et conçu pour se détacher de l'implant lors de la translation proximale du fil de traction.

9. Système selon la revendication 8, dans lequel l'élément de détachement comprend en outre une ouverture distale (24a, 24c, 24d) à travers la partie distale, et
dans lequel la fibre résistante à l'étirement passe à travers l'ouverture distale.

10. Système selon la revendication 9, dans lequel l'ouverture distale comprend une surface atraumatique (25) apposée à la fibre résistante à l'étirement.

11. Système selon la revendication 8, comprenant en outre :
un fil de boucle (400) fixé au tube de pose,
dans lequel l'élément de détachement comprend en outre une ouverture proximale (22a, 22c, 22d) à travers la partie proximale, et
dans lequel le fil de boucle est étendu à travers l'ouverture proximale et autour du fil de traction, de sorte que le tube d'administration est attaché à l'élément de détachement.

12. Système selon la revendication 11, dans lequel l'ouverture proximale comprend une surface atraumatique (23) apposée au fil de boucle, et éventuellement :
dans lequel la surface atraumatique comprend deux coins approfondis (27), chacun s'étendant respectivement dans une direction proximale par rapport au côté de l'ouverture atraumatique qui est orthogonal à l'axe longitudinal de l'élément de détachement, et
dans lequel le fil de boucle est positionné dans chacun des deux coins approfondis.

13. Système selon la revendication 11, dans lequel l'ouverture proximale a une forme proche d'un polygone à cinq côtés.

14. Système selon la revendication 8,
dans lequel l'élément de détachement comprend en outre une ouverture distale (24a, 24c, 24d) à travers la partie distale,
dans lequel l'élément de détachement comprend en outre une ouverture proximale (22a, 22c, 22d) à travers la partie proximale,
dans lequel l'élément de détachement comprend en outre un pont (28, 28a, 28c, 28d) séparant l'ouverture distale et l'ouverture proximale, et
dans lequel le pont supporte une partie du fil de traction.

15. Système selon la revendication 8, dans lequel (i) la partie proximale de l'élément de détachement comprend une largeur approximativement égale à un diamètre externe du tube de pose, (ii) la partie proximale de l'élément de détachement comprend une largeur approximativement égale à un diamètre externe de la spire métallique pour embolisation, ou (iii) la partie proximale comprend une extension proximale (38) comprenant une largeur inférieure et approximativement égale à un diamètre d'une lumière du tube de pose au niveau d'une extrémité distale du tube de pose.
